(19) Europäisches Patentamt
European Patent Office
Office européen des brevets

(11) Publication number: **0 487 756 A1**

# (12) EUROPEAN PATENT APPLICATION

(21) Application number: **90122476.6**

(22) Date of filing: **26.11.90**

(51) Int. Cl.[5]: **C07K 5/02**, C12P 21/02, A61K 37/02, //(C12P21/02, C12R1:51)

(43) Date of publication of application: **03.06.92 Bulletin 92/23**

(84) Designated Contracting States: **AT BE CH DE DK ES FR GB GR IT LI LU NL SE**

(71) Applicant: **HOECHST AKTIENGESELLSCHAFT Postfach 80 03 20 W-6230 Frankfurt am Main 80(DE)**

(72) Inventor: **Nadkarni, Suresh Rudra 5, Yagneshwar Kasturba Marg Mulund (west) Bombay 400080(IN)**
Inventor: **Chatterjee, Sugata H 1/2 Hoechst Ouarters Darga Road Mulund (West) Bombay 400082(IN)**
Inventor: **Patel, Mahesh Vithalbhai 1/Nandja B.P. Cross Road 2 Mulund (West) Bombay 400080(IN)**
Inventor: **Desikan, Kalyanpuram Rajgopalan 4, Krishna Kunj Opp Johnson L.B.S. Marg Muland (West) Bombay 400080(IN)**
Inventor: **Ganguli, Birnal Naresh 401-402 Vaikunth 8th Road Sindhi Society Chembur Bombay 400071(IN)**
Inventor: **Fehlhaber, Hans Wolfram Thomas-Mann-Strasse 5a W-6270 Idstein/Taunus(DE)**
Inventor: **Rupp, Richard Helmut Roederweg 16a W-6240 Königstein/Taunus(DE)**

(54) **Antibiotics napsamycins A-D, process for their production and their use as pharmaceuticals.**

(57) This invention relates to new antibacterial antibiotics Napsamycins A - D of the formula

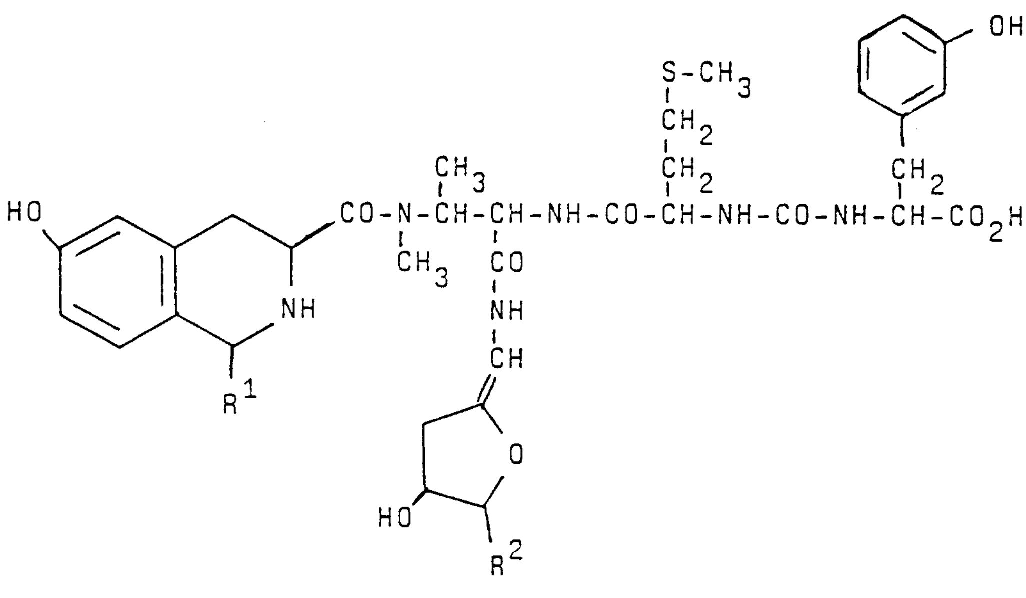



Rank Xerox (UK) Business Services

Napsamycin A : $R^1$ = H, $R^2$ = uracil
Napsamycin B : $R^1$ = $CH_3$, $R^2$ = uracil
Napsamycin C : $R^1$ = H, $R^2$ = dihydrouracil
Napsamycin D : $R^1$ = $CH_3$, $R^2$ = dihydrouracil
from a strain of Streptomyces candidus, Y-82,11372 (DSM 5940).

This invention relates to new antibacterial antibiotics named napsamycins A - D, from a strain of Streptomyces candidus, Y-82,11372 (Culture Number Hoechst India Limited Y-82,11372). The Napsamycins have the following chemical structures

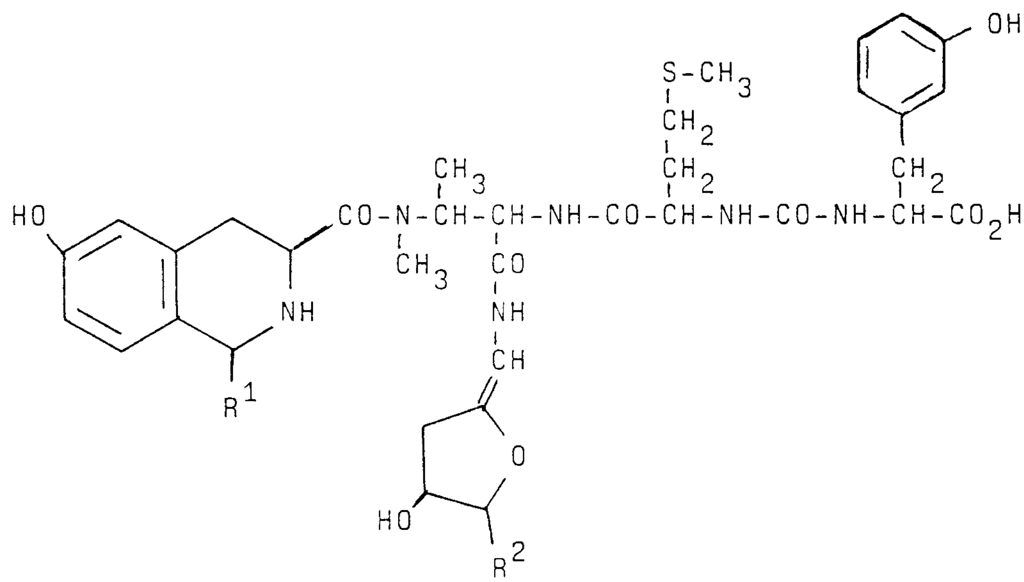

Napsamycin A : $R^1$ = H, $R^2$ = uracil
Napsamycin B : $R^1$ = $CH_3$, $R^2$ = uracil
Napsamycin C : $R^1$ = H, $R^2$ = dihydrouracil
Napsamycin D : $R^1$ = $CH_3$, $R^2$ = dihydrouracil

and are members of the recently discovered class of nucleoside peptide antibiotics. Up to now there are eleven structural variants known in this class of compounds, i.e. the Pacidomycins 1-7 (J. Antibiotics 1989, pp. 506-526) and the Mureidomycins A-D (J. Antibiotics 1989, pp. 662-679). All of them are different from Napsamycins A - D, especially with respect to the N-terminal 6-hydroxy-tetrahydroisoquinoline-3-carboxylic acid moiety and the 1-methyl-6-hydroxy-tetrahydroisoquinoline-3-carboxylic acid moiety, which are unique in Napsamycins A and C and Napsamycins B and D, respectively.

The Streptomyces candidus, culture No. Hoechst India Limited Y-82,11372, henceforward referred to as Y-82,11372, used for the production of napsamycins was isolated from a soil sample collected at Andaman Islands, India and was identified as Streptomyces candidus. The microorganism Y-82,11372 has been deposited with Deutsche Sammlung von Mikroorganismen under the conditions of the Treaty of Budapest on May 17, 1990 and it has received No. DSM 5940.

A further aspect of the present invention is to provide a process for the production of new antibacterial antibiotics napsamycins A - D from culture No. Hoechst India Limited Y-82,11372, its mutants or variants. Said process comprises cultivating streptomyces candidus Y-82,11372, its mutants or variants under aerobic conditions in a nutrient medium containing sources of carbon and nitrogen, nutrient inorganic salts and trace elements and isolating and purifying the said antibiotics from the culture broth.

The nutrient medium includes carbon sources such as starch, glucose, sucrose, dextrin, fructose, molasses, lactose or galactose. The preferred carbon source is starch. The nutrients medium also includes nitrogen sources such as soyabean meal, peanut meal, yeast extract, beef extract, peptone, malt extract, corn steep liquor or casamino acids. The preferred nitrogen sources are soyabean meal and yeast extract. The nutrient medium also includes nutrient inorganic salts such as sodium hydrogen phosphate, potassium hydrogen phosphate, sodium chloride, calcium chloride, calcium carbonate or magnesium sulphate. The nutrient medium also includes trace elements such as salts of iron, manganese, copper, zinc or cobalt or of such other heavy metals.

Cultivation of culture No. Y-82,11372 is preferably carried out at temperatures between 24-32 °C and pH between 6.0 to 8.0. In particular culture No. Y-82,11372 is cultivated at 28°C (± 1°C) and pH 6.0.

The cultivation is carried out for 60 to 72 hours when optimal yield of the antibiotics of the present invention is obtained. The cultivation is preferably carried out for 68 hours under submerged condition in shake flasks as well as in laboratory fermenters. If desired, the cultivation is carried out in fermenters in the presence of an antifoam agent such as ®Desmophen (Polyols, Bayer AG, Leverkusen, West Germany). The

progress of the cultivation and formation of napsamycins of the present invention can be detected by measuring the bioactivity of the culture broth against Pseudomonas species by the known agar plate diffusion assay method. The preferred culture is Pseudomonas aeruginosa.

In the resulting culture broth, napsamycins are present only in the culture filtrate which is separated from the mycelium by centrifugation. Napsamycins can for example be recovered from the culture filtrate by one or several known methods such as hydrophobic interaction chromatography on ®Diaion HP-20 (high porosity resin based on a polystyrene-divinylbenzene copolymer, Mitsubishi Chemicals Industries, Japan) or ®Amberlite XAD-2 (porous resin based on polystyrene-acrylic acid ester, Rohm & Haas Co., USA); or extraction with water immiscible solvents like butanol; or ion-exchange chromatography on ®Amberlite IRC-50 ($H^+$), "CG-50 II ($H^+$)" or SP-®Sephadex ($Na^+$). The preferred method is adsorption on ®Diaion HP-20 followed by desorption of the compound with suitable organic solvents such as methanol, acetonitrile and aqueous combination of these solvents. The preferred solvent for elution is aqueous methanol. Removal of the solvent from the active eluates gives crude napsamycins. Further purification can be achieved by chromatography on substances like silica gel, modified silica gel, cellulose or ®Sephadex LH-20 (gel of defined porosity on agarose, Pharmacia Fine Chemicals AG, Sweden). The preferred method is to chromatograph the crude napsamycins on silica gel using ethyl acetate - ammoniacal methanol mixture for elution by stepwise increase of the methanol concentration by 5-10 % at each step. The active eluates (monitored by bioassay) are concentrated to give the semi-pure compound. The semi-pure antibiotic thus obtained can be further purified by droplet counter current chromatography using an appropriate combination of organic solvents (like 2-propanol, methanol, chloroform) and water at controlled pH; or by chromatography on ®Sephadex G-10 or G-25; or on modified silica gel like octadecyldimethyl silyl silica (RP-18). The preferred method is to chromatograph the semi-pure antibiotic on RP-18 silica gel using methanol-water mixture for elution by stepwise increase of the methanol concentration by 5-10 % at each step. The active eluates (measured by bioassay) are concentrated to give a mixture of Napsamycins A - D. Separation of the two compounds can be achieved by preparative HPLC (high pressure liquid chromatography) on a modified silica gel column using solvents such as $CH_3OH$, $CH_3CN$, acetone, water or appropriate combinations thereof. The preferred method is preparative HPLC on an ®ODS-hypersil column using a 40:60 mixture of a methanol and aqueous sodium phosphate buffer at pH 7.2. The separated components are individually concentrated under reduced pressure to remove methanol. The resulting aqueous solutions are then desalted by ®Diaion HP-20 chromatography using methanol, $CH_3CN$, acetone, water and appropriate combinations thereof. The preferred eluent is aqueous methanol. Removal of the organic solvent followed by lyophilization to remove water gives one fraction containing Napsamycin A plus Napsamycin C (20 - 45% according to FAB-MS) and a second fraction containing Napsamycin B plus Napsamycin D (20 - 45% according to FAB-MS).

These mixtures of pure antibiotics are white amorphous powders which are well soluble in water and aqueous methanol. They exhibit no definite melting points (> 190 °C decomposition), and show an UV absorption maximum at 256 nm (in water). The single components of the mixtures are characterized by Fast Atom Bombardment (FAB) and Collisional Induced Decomposition (CID) mass spectra:

Napsamycin A: molecular formula $C_{39}H_{48}N_8O_{12}S$;
high-resolution FAB-MS,
$M + H^+$ found m/z 853.3192;
$M + H^+$ calculated m/z 853.3191;
MS/MS (CID Argon), see figure 1 of the accompanying drawings.

Napsamycin B: molecular formula $C_{40}H_{50}N_8O_{12}S$;
high-resolution FAB-MS,
$M + H^+$ found m/z 867.3347,
$M + H^+$ calculated m/z 867.3347;
MS/MS (CID Argon), see figure 2 of the accompanying drawings.

Napsamycin C: molecular formula $C_{39}H_{50}N_8O_{12}S$;
high-resolution FAB-MS,
$M + H^+$ found m/z 855.3330,
$M + H^+$ calculated m/z 855.3347;
MS/MS (CID Argon), see figure 3 of the accompanying drawings.

Napsamycin D: molecular formula $C_{40}H_{52}N_8O_{12}S$;
high-resolution FAB-MS,
$M + H^+$ found m/z 869.3477,
$M + H^+$ calculated m/z 869.3504;
MS/MS (CID Argon), see figure 4 of the accompanying drawings.

After hydrolysis with 6N HCl at 110°C for 20 hrs. in a sealed tube, esterification of the hydrolysis products with methanolic HCl and subsequent N.O-trifluoroacetylationwith trifluoroacetic acid anhydride, a GC/MS analysis showed the presence of the appropriate derivatives of the following amino acids:

2-amino-3-methylamino-butyric acid,
5-amino-4-oxo-pentanoic acid,
$\beta$-alanine (cleavage product of dihydrouracil),
methionine,
meta-tyrosine,
6-hydroxy-tetrahydroisoquinoline-3-carboxylic acid (Napsamycins A + C only),
6-hydroxy-1-methyl-tetrahydroisoquinoline-3-carboxylic acid (Napsamycins B + D only).

Napsamycins A - D and their physiologically tolerated salts can be administered, for example, orally, intramuscularly or intravenously. Pharmaceuticals which contain Napsamycins as active substance are subject of the present invention also. They can be prepared by mixing the said compound with one or more pharmacologically tolerated auxiliaries and/or excipients such as, for example, fillers, emulsifiers, lubricants, masking flavours, colorants or buffer substances, and converted into a suitable pharmaceutical form such as, for example, tablets, coated tablets, capsules, or a suspension or solution suitable for parenteral administration. Examples of auxiliaries and/or excipients which may be mentioned are tragacanth, lactose, talc, agar-agar, polyglycols, ethanol and water. Suitable and preferred for parenteral administration are suspension or solutions in water. It is also possible to administer the active substances as such, without vehicles or diluents, in a suitable form, for example in capsules.

Suitable doses of the compound of this invention or its physiologically tolerated acid addition salts are about 0.1 to 20 g/day, preferably 0.5 to 4 g/day, for an adult of body weight about 60 kg.

It is possible to administer single doses or, in general, multiple doses, it being possible for the single dose to contain the active substance in an amount of about 50 to 4000 mg, preferably of about 500 to 2000 mg.

The following examples are illustrative of the present invention:

Example 1

Isolation of the culture Y-82,11372 from soil

(a) Composition of nutrient isolation medium.

| | |
|---|---|
| Corn Starch | 10.0 g |
| Casein | 1.0 g |
| Peptone | 1.0 g |
| Beef extract | 1.0 g |
| $K_2HPO_4$ | 0.5 g |
| Agar powder | 13.0 g |
| Demineralized water | 1000.0 ml |
| pH | 7.5 |

(b) Soil planting and isolation

10 g of soil collected from Andaman Islands were added to 90 ml of sterilized demineralized water in a 250 ml Erlenmeyer flask which was shaken for 2 hours on a rotary shaker (220 rpm). The above soil suspension was then serially diluted in steps of 10 upto $10^{-5}$. From the last dilution, 1 ml of suspension was placed at the centre of a sterile glass petri plate (15 cms diameter) to which was then poured approximately 50 ml of the above isolation medium cooled to 45 °C and the plate swirled thoroughly. The mixture of soil suspension and medium was allowed to settle and incubated at 28 °C (± 1 °C) for 5 days. The petri plate was periodically observed and the culture No. Y-82,11372 was isolated from amongst the growing microorganisms.

Example 2

Maintenance of the culture Y-82,11372

Composition of maintenance medium

Culture No. Y-82,11372 was maintained on nutrient isolation medium described in Example 1.

After dissolving the ingredients thoroughly by heating, it was distributed in test tubes and then sterilized at 121 °C for 20 minutes. The test tubes were cooled and allowed to solidify in a slanting position. The agar slants were streaked with the growth of the culture No. Y-82,11372 by a wire loop and incubated at 28 °C (± 1 °C) until a good growth was observed. The well grown cultures were stored in the refrigerator at +8 °C.

Example 3

Fermentation of culture Y-82,11372 in shake flasks

Composition of seed culture medium

| | |
|---|---|
| Soluble starch | 24.0 g |
| Glucose | 1.0 g |
| Beef Extract | 3.0 g |
| Tryptone | 5.0 g |
| Soyabean meal | 5.0 g |
| Yeast extract | 5.0 g |
| $CaCO_3$ | 1.0 g |
| Demineralised water | 1000.0 ml |
| pH | 6.0 |

The above seed culture medium was distributed in 75 ml amounts in 500 ml Erlenmeyer flasks and autoclaved at 121 °C for 20 minutes. The flasks were cooled and each was inoculated with a loopful of the above mentioned well grown culture of example 2 and shaken for 24 hours at 220 rpm at 28 °C (± 1 °C). This was used as seed culture for inoculating the production medium flasks as discussed below.

Production of the antibiotics napsamycins in shake flasks

Composition of the production medium

| | |
|---|---|
| Soluble starch | 25.0 g |
| Glucose | 10.0 g |
| Yeast extract | 2.0 g |
| Soyabean meal | 20.0 g |
| NaCl | 5.0 g |
| $MgSO_4 \cdot 7H_2O$ | 1.0 g |
| Trace elements solution | 1.0 g |
| Demineralized water | 1000.0 ml |
| pH | 6.0 |

Trace elements solution

| | |
|---|---|
| $CuSO_4 \cdot 5H_2O$ | 7.0 g |
| $FeSO_4 \cdot 7H_2O$ | 1.0 g |
| $MnCl \cdot 4H_2O$ | 8.0 g |
| $ZnSO_4 \cdot 7H_2O$ | 2.0 g |
| Demineralized water | 1000.0 ml |

The production medium was distributed in 60 ml amounts in 500 ml Erlenmeyer flasks and autoclaved at 121 °C for 20 minutes. The flasks were cooled and then inoculated with the above mentioned seed culture (1 % v/v). The fermentation was carried out on a rotary shaker at 220 rpm and at a temperatur of 28 °C (± 1 °C) for 68 hours.

The production of the antibiotic was determined by the bioactivity profile tested against Pseudomonas aeruginosa, using the well diffusion method in a known manner. After harvesting the culture broth was centrifuged and napsamycin were isolated from the culture filtrate and purified as described herein below.

Example 4

Cultivation of the culture No. Y-82,11372 in fermenters

Preparation of seed culture in Shake Flasks

The seed culture medium of Example 3 (75 ml) was taken in 500 ml Erlenmeyer flasks with presterilization pH adjusted to 6.0. This was sterilized in an autoclave at 121 °C for 22 minutes, cooled and inoculated with a loopful of the well grown culture of Example 2. The flasks were incubated at 28 °C (± 1 °C) for 24 hours at 220 rpm in a rotary shaker. This growth culture was used to inoculate fermenters as outlined below.

Large Scale Fermentation

90 litres of the production medium (same as production medium mentioned in Example 3) in 150 litre Marubishi fermenter with presterilization pH adjusted to 6.0 was sterilized in situ for 28 minutes at 121 °C, and seeded with 3 litres of the pooled seed mentioned above.

These were run with the following parameters:

| | |
|---|---|
| Temperatur : | 28 °C (± 1 °C) |
| Agitation : | 80-100 r.p.m. |
| Aeration : | 80 litres per minute |
| Harvest time : | 68-70 hours |

®Desmophen was added as the antifoam during fermentation by automatic control through AF probe.

The production of the antibiotic was determined by the activity profile tested against Pseudomonas aeruginosa. When fermentation was discontinued, the pH of the culture broth was 6.5. The culture broth was centrifuged after harvesting and the antibiotic was isolated and purified from the culture filtrate as mentioned herein below.

Approximately 250 litres of the harvested broth were separated from the mycelium by centrifugation. The resulting filtrate (pH 5.6-7.0) was passed through a column 14 l of ®Diaion HP-20. The column was washed with 40 l of demineralized water. It was then eluted with 60 l of water. The process was repeated till the water washings were colourless. The column was then eluted with 30 l of water of which the pH was adjusted to 3.5-4.0 with concentrated HCl and then washed with 30 l of demineralized water. Elutions were then carried out using 30 l each of 10:90, 20:80, 30:70, 40:60 and 50:50 mixture of methanol and water. The eluates were collected in fractions of 2 l size. The napsamycins were eluted out (as monitored by bioactivity against Pseudomonas aeruginosa) by both 30:70 and 40:60 mixture of methanol and water. The active fractions were combined to a total volume of 60 l, concentrated under reduced pressure to remove MeOH and then lyophilized to afford 38 g of a mixture of crude napsamycin A and B as a brown powder.

Crude napsamycins were then subjected to medium pressure liquid chromatography (MPLC) over 2.8 kg of silica gel (250-400 mesh) packed in a 7.5 cm x 129 cm glass column. The column was developed in ethyl acetate using a flow rate of 40-50 ml $min^{-1}$, and then eluted with 15 l of a 80:20 mixture of ethyl acetate and 1 % (v/v) ammoniacal methanol, 10 l of a 75:25 mixture, 12 l of a 70:30 and 10 l of a 65:35 mixture. Further elutions were carried out using 10 l of a 60:40 mixture, 17 l of a 50:50 and 15 l of a 40:60 mixture of EtOAc and 1 % (v/v) ammoniacal methanol. The eluates were collected in 1.5 l fractions and

monitored by bioassay. Napsamycins eluted out in 60:40 mixture of ethyl acetate and 1 % ammoniacal methanol. The active eluates were combined and the solvents removed under vacuum to give 11.6 g of semi-pure napsamycins as a yellowish brown powder.

Further purification of the semi-pure napsamycins thus obtained was carried out by MPLC on 600 g of RP-18 silica gel (ca. 55 µ) packed in a 5.5 cm x 63 cm glass column. The column was developed in 10 l of a 15:85 mixture of MeOH and water. The column was successively eluted with 6 l of a 25:75 mixture, 3.5 l of a 30:70 mixture, 2 l of a 40:60 mixture of methanol and water, and finally with 4 l of methanol. The eluates were collected at a flow rate of 40 ml $min^{-1}$ into fractions of 500 ml size. The fractions were monitored by both microbial assay and by UV detection at 220 nm. Napsamycins eluted out with the 30:70 mixture of methanol and water. Methanol was removed under reduced pressure and water was lyophilized to afford 1.136 g of a mixture of napsamycins A - D as a pale yellow powder.

The mixture of napsamycins A - D was dissolved in 113 ml of a 40:60 mixture of methanol and water and was then subjected to preparative high pressure liquid chromatography (HPLC) on a 1.6 cm x 120 cm ®ODS-hypersil (10 µ) column using an eluant of a 40:60 mixture of methanol and 0.02 M aqueous sodium phosphate buffer (pH 7.2) at a flow rate of 1.4 ml $min^{-1}$ with a UV detection set at 220 nm using a chart speed of 0.5 cm $min^{-1}$. In each injection 5 mg (0.5 ml) of the sample mixture was loaded. Under these conditions, Napsamycins A + C had a retention time of 4.4 mins and Napsamycins B + D eluted at 5.2 min. Napsamycins A + C and B + D eluted out in total volumes of 150 ml and 200 ml respectively.

Desalting of the napsamycins was carried out in the following way. The HPLC eluate corresponding to this compound was concentrated under vacuo to remove methanol and the resulting aqueous solution was diluted with equal volume of double distilled water. This solution was passed through a column of 130 ml of ®Diaion HP-20 and washed with double distilled water till the washings did not respond to a phosphate analysis by ammonium molybdate method. The column was eluted with 50 : 50 mixture of MeOH and double distilled water till the eluates did not show any microbial activity. Removal of MeOH under reduced pressure followed by lyophilization afforded the pure napsamycins as white powder.

The purity of the napsamycins was checked on a 4 x 250 mm ®ODS-hypersil (10 µ) column; eluant 40 : 60 mixture of MeOH and 0.02 M aqueous sodium phosphate buffer (pH 7.2); flow rate 0.5 ml $min^{-1}$; chart speed 10 mm $min^{-1}$; detection at 220 nm: Napsamycins A + C eluted at 5.8 min and Napsamycins B + D at 6.8 min respectively.

Napsamycins A - D are essentially active against pseudomonas species with MIC-values in the range from 3 - 50 µg/ml.

## Claims

1. Napsamycin A, a compound of the formula $C_{39}H_{48}N_8O_{12}S$, Napsamycin B, a compound of the formula $C_{40}H_{50}N_8O_{12}S$, Napsamycin C, a compound of the formula $C_{39}H_{50}N_8O_{12}S$, and/or Napsamycin D, a compound of the formula $C_{40}H_{52}N_8O_{12}S$, obtained by Streptomyces candidus species Y-82,11372 (DSM 5940) producing migroorganism and the subsequent isolation and purification from the culture broth, as well as the pharmaceutically acceptable salts thereof.

2. A process for the preparation of Napsamycins A - D as claimed in claim 1, which comprises Streptomyces candidus species Y-82,11372 (DSM 5940), the mutants and/or variants thereof, being cultivated under aerobic conditions in a nutrient medium containing sources of carbon and of nitrogen, inorganic nutrient salts and trace elements, and the compound being isolated and purified from the culture broth in a customary manner.

3. The process as claimed in claim 2, wherein the cultivation is carried out at temperatures between 24 and 32 °C and a pH between about 6.0 to 8.0.

4. The process as claimed in claims 2 or 3, wherein the cultivation is carried out at 28 °C (± 1 °C) and a pH about 6.0.

5. The process as claimed in one or more of claims 2 to 4, wherein the fermentation is carried out for 68 to 72 hours.

6. The process as claimed in one or more of claims 2 to 5, wherein the fermentation is carried out as submerged fermentation.

7. A pharmaceutical which contains a compound as claimed in claim 1 where appropriate in additon to auxiliaries and/or excipients customary for the preparation of pharmaceuticals.

8. The use of a compound as claimed in claim 1 for the preparation of pharmaceuticals having an antibiotic action.

9. The use of a compound as claimed in claim 1 having antibacterial antibiotic action.

10. Streptomyces candidus species Y-82,11372 (DSM 5940).

MS/MS Spectrum of Napsamycin A

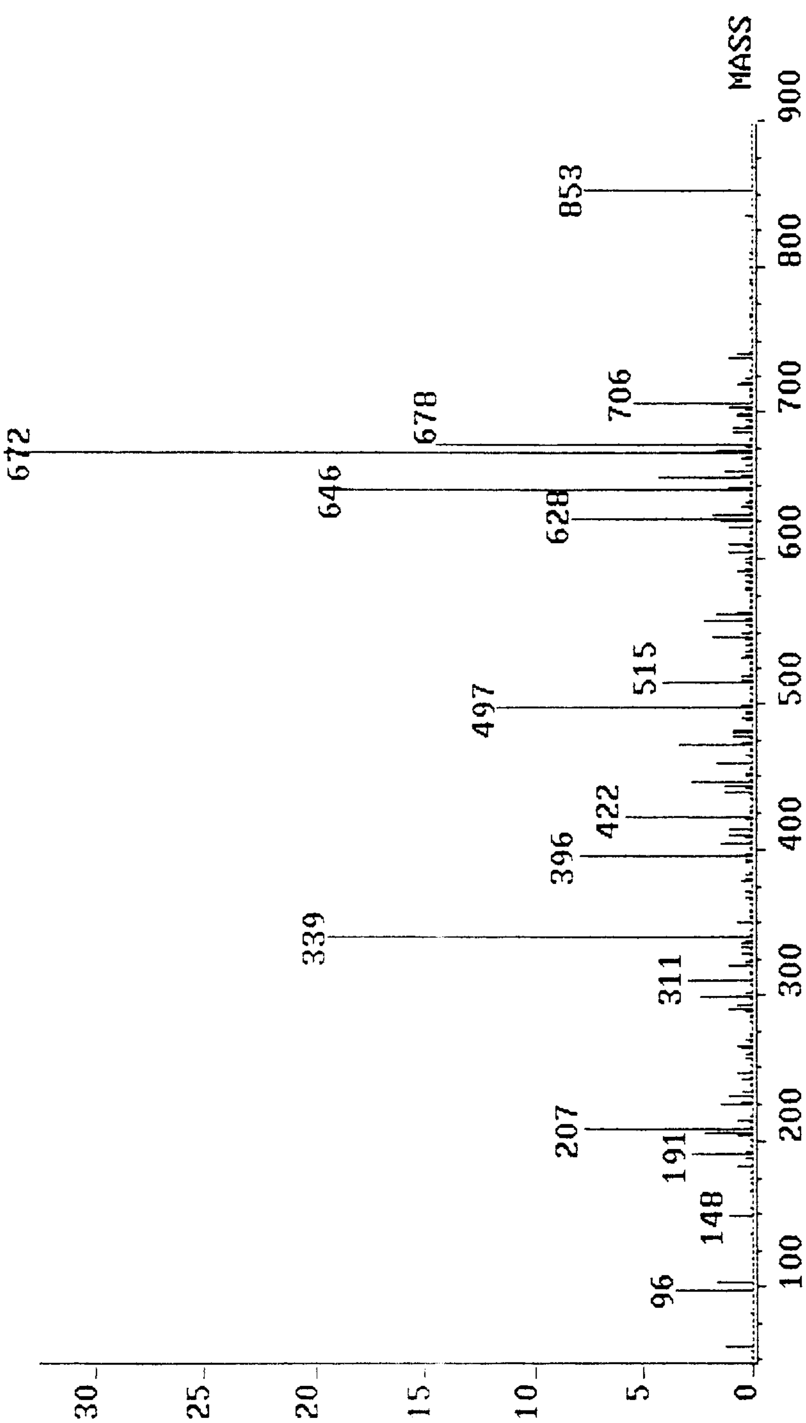


*Figure 1*

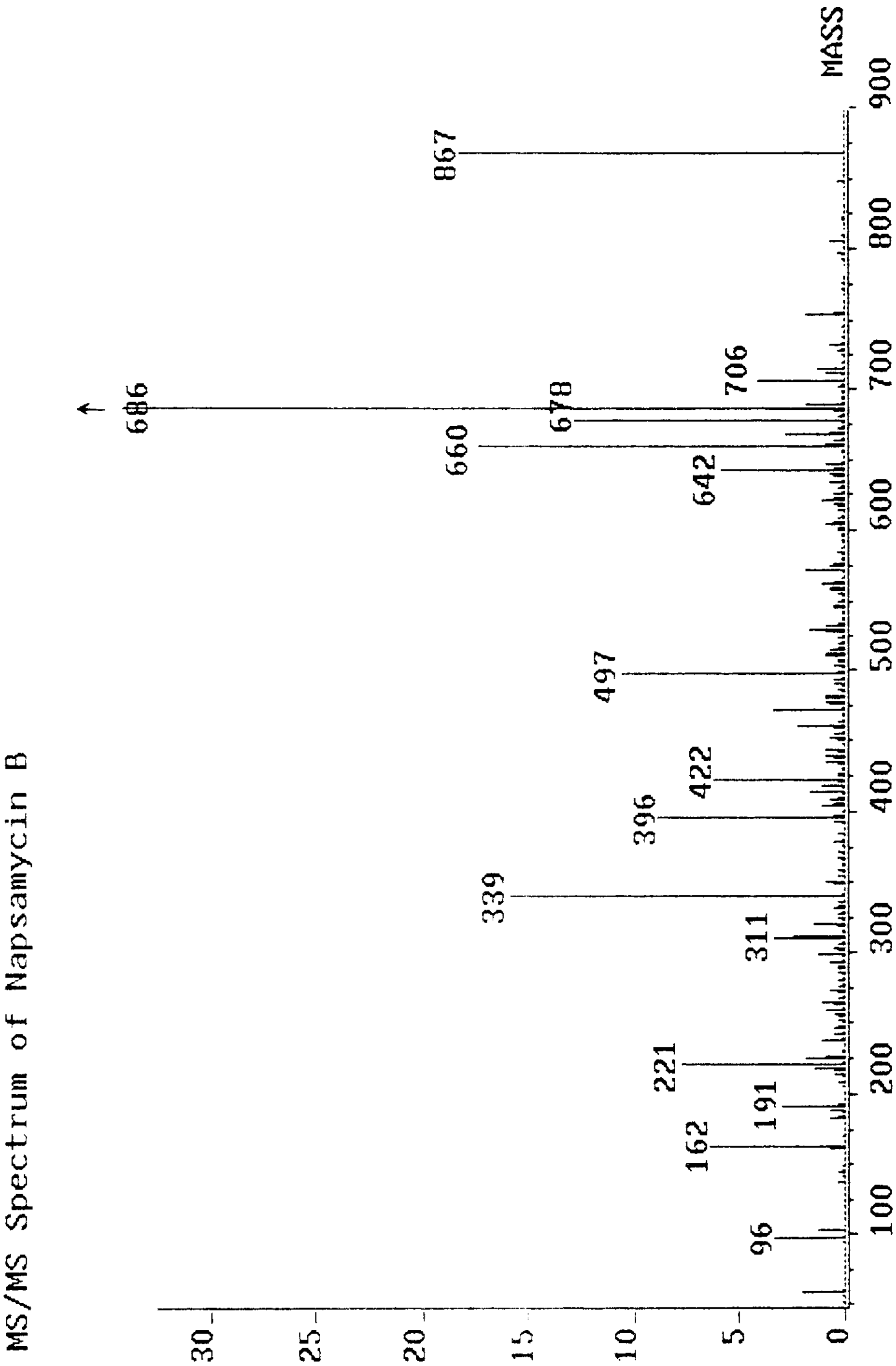
MS/MS Spectrum of Napsamycin B
30
25
20
15
10
5
0
100
200
300
400
500
600
700
800
900
MASS
96
162
191
221
311
339
396
422
497
642
660
678
686
706
867

*Figure 2*

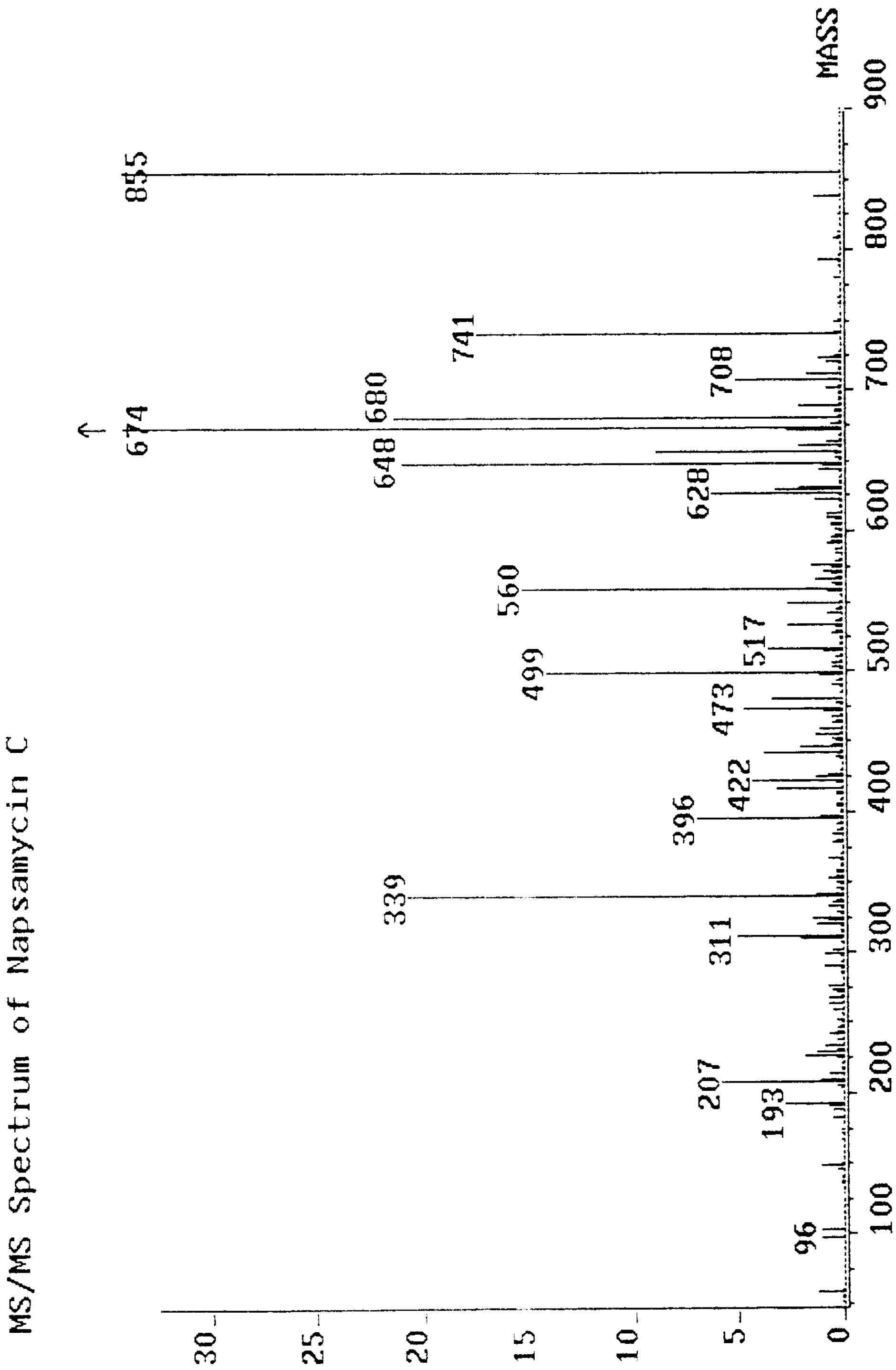
MS/MS Spectrum of Napsamycin C
30
25
20
15
10
5
0
100
200
300
400
500
600
700
800
900
MASS
96
193
207
311
339
396
422
473
499
517
560
628
648
674
680
708
741
855

*Figure 3*

MS/MS Spectrum of Napsamycin D

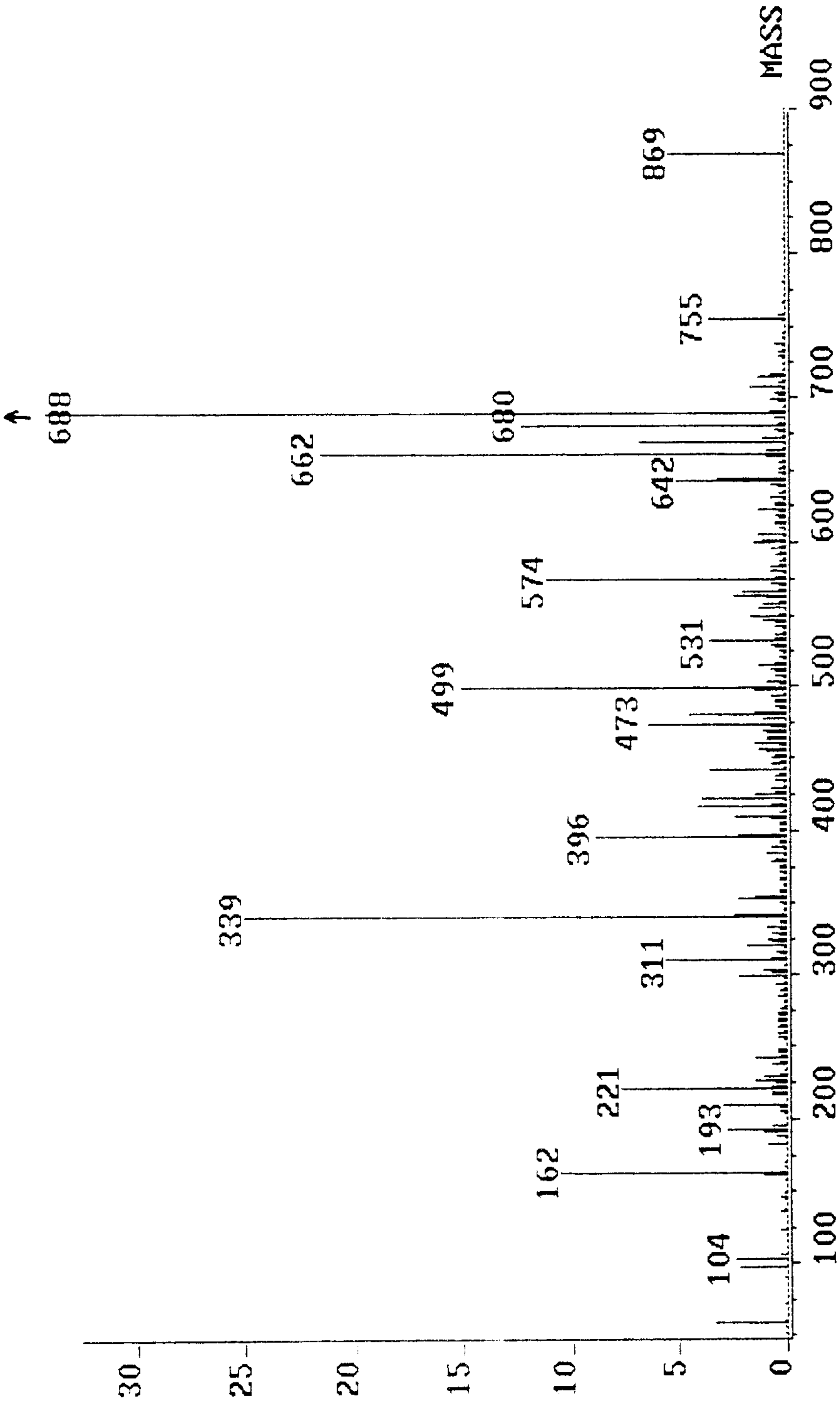


*Figure 4*

European Patent Office

# PARTIAL EUROPEAN SEARCH REPORT

which under Rule 45 of the European Patent Convention shall be considered, for the purposes of subsequent proceedings, as the European search report

Application number
EP 90 12 2476

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 317 292 (SANKYO CO. LTD)<br>* Page 2, line 1 - page 5, line 15; page 12, lines 12-38; page 13, line 45 -page 14, line 48; examples 1-4,6,8; claims 1-4,6, 8,9,12,14 *<br>---- | 1-8 | C 07 K 5/02<br>C 12 P 21/02<br>A 61 K 37/02//<br>(C 12 P 21/02<br>C 12 R 1:51) |

TECHNICAL FIELDS SEARCHED (Int. Cl.4)

C 07 K
C 12 P
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely: 9
Claims not searched:
Reason for the limitation of the search:

Claim 9 has not been searched as far as it concerns subject matter excluded from patentability.

Method for treatment of the human or animal body by surgery or therapy (see Art. 52(4) of the European Patent Convention).

| Place of search | Date of completion of the search | Examiner |
|---|---|---|
| THE HAGUE | 17-07-1991 | GROENENDIJK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document